# EUROPEAN PATENT APPLICATION

(11) **EP 4 177 831 A1**
(43) Date of publication of application: **10.05.2023**
(21) Application number: 21206185.7
(22) Date of filing: 03.11.2021
(51) Int. Cl.: G06T 7/10, G06T 7/68, G06T 19/20, G06V 40/16, G09B 19/00, G16H 20/30, G06F 3/01, B26B 19/46, B26B 21/40

(54) **ASSISTING A PERSON TO PERFORM A PERSONAL CARE ACTIVITY**

(71) Applicant: Koninklijke Philips N.V., 5656 AG Eindhoven (NL)
(72) Inventor: FLINSENBERG, Ingrid ,Christina, Maria, Eindhoven (NL); ZNAMENSKIY, Dmitry, Nikolaevich, Eindhoven (NL); GALLUCCI, Alessio, Eindhoven (NL); SCHUIJERS, Erik, Gosuinus, Petrus, Eindhoven (NL)
(74) Representative: Philips Intellectual Property & Standards

(57) **Abstract**

According to an aspect, there is provided a computer-implemented method (100) for assisting a subject to perform a personal care activity. The method (100) comprises receiving (102) image data relating to at least part of a subject's head; creating (104) a spatial representation of the subject's head by mapping the received image data to a model template having a first side and a second side; detecting (106) a style feature in the spatial representation; determining (108) a parameter of the style feature; generating (110) a symmetrised spatial representation comprising at least one of: a symmetrised first side that is a symmetrised version of the second side of the spatial representation; and a symmetrised second side that is a symmetrised version of the first side of the spatial representation; comparing (112) the parameter of the style feature in the symmetrised spatial representation with the parameter of the style feature in the spatial representation; and generating (114), based on the comparison, a delta representation for presentation to the subject, the delta representation including an indication of a difference between the parameter of the style feature in the symmetrised spatial representation with the parameter of the style feature in the spatial representation.

## Description

### FIELD OF THE INVENTION

The invention relates to personal care activities and, more particularly, to methods and systems that assist a person to perform such an activity.

### BACKGROUND OF THE INVENTION

When performing a personal care activity, such as cutting or trimming hair on one's head or face (e.g., shaving), it is often desirable to perform the activity equally on both sides of the face or head, so that the result is symmetrical. For example, a person may style their hair in a particular way, apply make-up or face paint, or trim or shave their facial hair, and in each case, it may be intended that symmetrical styling is achieved.

Achieving a symmetrical style when performing the styling activity using just one's reflection in a mirror can be difficult, particularly if the person needs to rotate their head in order to see the part of their head being styled. There is, therefore, a desire for a system that can assist a person performing a personal care activity or a styling activity, when it is intended that the result appears symmetrical.

### SUMMARY OF THE INVENTION

The inventors of the present invention have recognised that, in order to assist a person in performing a personal care (e.g., styling) activity, guidance may be provided that indicates to the person how symmetrical the style is so that the person can take action to improve the symmetry. Further guidance may be provided to indicate to the person what action is to be taken to improve the symmetry of the style.

According to a first specific aspect, there is provided a computer-implemented method for assisting a subject to perform a personal care activity. The method comprises receiving image data relating to at least part of a subject's head; creating a spatial representation of the subject's head by mapping the received image data to a model template having a first side and a second side; detecting a style feature in the spatial representation; determining a parameter of the style feature; generating a symmetrised spatial representation comprising at least one: a symmetrised first side that is a symmetrised version of the second side of the spatial representation; and a symmetrised second side that is a symmetrised version of the first side of the spatial representation; comparing the parameter of the style feature in the symmetrised spatial representation with the parameter of the style feature in the spatial representation; and generating, based on the comparison, a delta representation for presentation to the subject, the delta representation including an indication of a difference between the parameter of the style feature in the symmetrised spatial representation with the parameter of the style feature in the spatial representation.

In some embodiments, the computer-implemented method may further comprise providing the delta representation for presentation on a representation of the head of the subject. The representation of the head of the subject may comprise a representation selected from a group comprising: an image formed using the received image data; an avatar; a generic image of a head; and a reflection of the head of the subject.

The computer-implemented method may further comprise generating a guidance indicator for presentation to the subject, the guidance indicator comprising an indication to guide the subject with regard to the performance of the personal care activity so as to reduce the difference between the parameter of the style feature in the symmetrised spatial representation with the parameter of the style feature in the spatial representation.

In some embodiments, the style feature may comprise a feature relating to a style of hair present on the subject's head. The personal care activity may comprise a hair cutting activity. The guidance indicator may comprise an indication to guide the subject to cut the hair in order of decreasing intended hair length.

The method may, in some embodiments, further comprise performing image segmentation in respect of the received image data to determine a first style feature and a second style feature. Determining a parameter of the style feature may comprise determining a style feature of each of the first style feature and the second style feature.

In some embodiments, the method may further comprise receiving further image data relating to at least part of the subject's head; updating the spatial representation of the subject's head based on the further image data; detecting the style feature in the updated spatial representation; determining an updated parameter of the style feature; generating an updated symmetrised spatial representation of the spatial representation; comparing the updated parameter of the style feature in the symmetrised spatial representation with the updated parameter of the style feature in the updated spatial representation; and generating, based on the comparison, an updated representation for presentation to the subject, the updated representation including an indication of a difference between the parameter of the style feature in the symmetrised spatial representation with the parameter of the style feature in the spatial representation.

The method may further comprise receiving location data indicative of a location of a personal care device used to perform the personal care activity. The method may further comprise providing an indication of the location of the personal care device for presentation with the representation to the subject.

In some embodiments, the method may comprise generating an instruction signal for delivery to a personal care device used to perform the personal care activity, the instruction signal configured to control an operating parameter of the personal care device to assist the user of the personal care device to reduce the difference between the parameter of the style feature in the symmetrised spatial representation with the parameter of the style feature in the spatial representation.

According to a second specific aspect, there is provided a computer program product comprising a non-transitory computer readable medium, the computer readable medium having computer readable code embodied therein, the computer readable code being configured such that, on execution by a suitable computer or processor, the computer or processor is caused to perform steps of the methods disclosed herein.

According to a third specific aspect, there is provided a system for assisting a subject to perform a personal care activity. The system comprises an imaging device configured to capture image data relating to at least part of a subject's head; a display; and a processor. The processor is configured to receive, from the imaging device, image data relating to at least part of a subject's head; create a spatial representation of the subject's head by mapping the received image data to a model template having a first side and a second side; detect a style feature in the spatial representation; determine a parameter of the style feature; generate a symmetrised spatial representation comprising at least one of: a symmetrised first side that is a symmetrised version of the second side of the spatial representation; and a symmetrised second side that is a symmetrised version of the first side of the spatial representation; compare the parameter of the style feature in the symmetrised spatial representation with the parameter of the style feature in the spatial representation; and generate, based on the comparison, a representation for presentation to the subject via the display, the representation including an indication of a difference between the parameter of the style feature in the symmetrised spatial representation with the parameter of the style feature in the spatial representation.

In some embodiments, the imaging device may be further configured to capture image data relating to a personal care device used to perform the personal care activity. The processor may be configured to determine, based on the captured image data relating to a personal care device, a location of a personal care device; and provide an indication of the location of the personal care device for presentation with the representation to the subject via the display.
The system may further comprise a personal care device for applying style features to the subject's head, the personal care device used to perform the personal care activity.

In some embodiments, the personal care device may comprise a sensor configured to measure at least one motion parameter indicative of a motion of the personal care device. The processor may be configured to determine a location of a personal care device based on the measured motion parameter.

The system may comprise a location beacon configured to be worn by the subject. The processor may be configured to determine a location of the personal care device relative to the location beacon.

In some embodiments, the personal care device may comprise a hair cutting device; the imaging device and the display may be components of at least one of: a personal electronic device; and an interactive mirror; the personal care activity may comprise a hair cutting activity; and/or the parameter of the style feature may comprise a length of hair on the subject's head.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a flowchart of an example of a method for assisting a subject to perform a personal care activity;
Fig. 2 is a collection of images showing representations that may be presented to a subject as part of the method;
Fig. 3 is a flowchart of an example of a further method for assisting a subject to perform a personal care activity;
Fig. 4 is a schematic illustration of an example of a processor in communication with a machine-readable medium;
Fig. 5 is a schematic illustration of an example of a system for assisting a subject to perform a personal care activity; and
Fig. 6 is a schematic illustration of a further example of a system for assisting a subject to perform a personal care activity.

### DETAILED DESCRIPTION OF THE EMBODIMENTS

Embodiments disclosed herein provide a mechanism by which a person performing a personal care activity can be assisted to help the person to perform the activity in a symmetrical manner with regard to their face or head. It can be difficult for a person performing such an activity on their own face or head using a mirror to ensure that the activities performed equally on both sides of their face/head, and failure to perform the activity symmetrically can lead to undesired results, leaving the person with negative feelings.

As used herein, the expression "personal care activity" is intended to include any care activity that a person might perform on themselves or that may be performed in respect of a person by somebody else. Examples of such personal care activities include hair styling, such as styling their hair on a person's head or face (e.g., trimming facial hair), facial styling, such as applying a style to a person's face using make up, face paint, or the like, or any other type of styling where it may be desirable to achieve a result on one side of the face or head that matches, reflects or corresponds to the results on the other side of the face or head. For example, it may be desirable when trimming one's moustache for both ends of the moustache to end at the same height relative to a person's mouth or nose. In another example, when applying eyeshadow, it may be desirable for the eyeshadow to be applied to the same extent both sides of the person's face.

Various embodiments disclosed herein may be performed using one or more processors of a computing device or computing system, including, for example, a smart phone, a tablet computer, a laptop computer, a desktop computer, a wearable device, an interactive mirror, or components of a cloud-based computing environment.

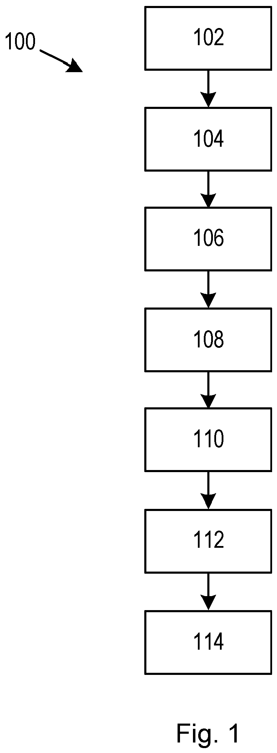

Referring now to the drawings, Fig. 1 is a flowchart of an example of a method 100, for example a method for assisting a subject to perform a personal care activity. The method 100 comprises, at step 102, receiving image data relating to at least part of a subject's head. The image data may, for example, comprise an image such as a photograph, a series of images or video footage, and the image data may be received from a storage medium (e.g., a memory) or from an image capture device such as a camera. While a single image is sufficient to enable the method 100 to be performed, multiple images may, in some embodiments, be received at step 102. For example, the received image data may include multiple images of the subject's head, each captured from a different angle (e.g., a photograph of the left side of the subject's head, a photograph of the right side of the subject's head, and a photograph of the front of the subject's head). In other examples, however, a single photograph of the front of the subject's head may be used. In examples where the received image data comprises image data taken from multiple images of the subject's head, image processing techniques may be used to "stitch" the images together, to form one large image. The image data may include at least part of the subject's face.

In some examples, the image data received at step 102 may be analysed according to one or more quality metrics and, if it is determined that the received image data fails to meet a quality metric, then a request may be issued for further and/or replacement image data. For example, if it is determined that the image data does not show enough of the subject's head, then a request may be made for one or more additional photographs showing more of the subject's head. Similarly, if it is determined that the image quality of the image data is insufficient (e.g., if the received image is blurry), then the subject may be requested to provide (e.g., capture or upload) a replacement image. In some examples, if the image data received at step 102 is sufficient (i.e., if the image data meets a defined quality metrics), then the subject may be informed by a message or through the use of an indicator (e.g., the display of a green color).

At step 104, the method 100 comprises creating a spatial representation of the subject's head by mapping the received image data to a model template having a first side and a second side. The model template may, for example, comprise a three-dimensional model template of a generic human head, which may be stored in a storage medium such as a memory, accessible by a processor used to performing the method 100. The model template may, for example, comprise a mesh template. Thus, in some examples, the spatial representation may comprise a three-dimensional model of the subject's head (or a part thereof). The spatial representation may, in some embodiments, be created using UV mapping techniques, in which a 2D image is projected to a 3D model's surface for texture mapping. Using such techniques, the image data (e.g., an image of the subject's face) may be separated into its geometry (e.g., its UV coordinated) and its texture.

In other examples, the spatial representation may be created using a three-dimensional head model (e.g., a parametric three-dimensional head model). In such examples, the representation of a three-dimensional mesh may be estimated, including parameters such as translation (e.g., position and distance) and orientation (e.g., pose). In this way, each pixel of the image data may be mapped onto a 3D coordinate of the mesh.

Other techniques may also be used for creating the spatial representation of the subject's head, including, for example, machine learning techniques, such as an artificial neural network model trained to generate such a spatial representation based on image data.

The method 100 comprises, at step 106, detecting a style feature in the spatial representation. Thus, once the spatial representation has been created at step 104, the spatial representation may be analysed using suitable processing techniques to detect more defined style features. A style feature may comprise a feature of the styling activity or personal care activity that has been or is to be performed, such as a hair style feature and/or a facial style feature (e.g., a make-up style feature). For example, a style feature may comprise a facial hair style feature, and step 106 of the method 100 may involve detecting regions of hair in the spatial representation and/or regions where hair is expected or likely to grow on the subject's head depicted in the spatial representation. In some examples, a user (e.g., the subject) may provide an indication of the style feature that is to be detected at step 106. For example, the subject may indicate that they are styling their facial hair, and this may lead to the detection of facial hair style features at step 106. In another example, the subject may indicate that they are performing make-up styling on their face, and as a result, the method 100 may detect, at step 106, features on the spatial representation that appeared to be applied make up, or regions of the spatial representation where it might be expected that make-up is to be applied. Again, in some examples, the subject may provide an indication of the type of make up to be applied, or an indication of the location where on the head or face the make-up is to be applied.

At step 108, the method 100 comprises determining a parameter of the style feature. For example, step 108 may involve determining a property of the face and/or head of the subject (e.g., a property of the subject's skin and/or a property of the subject's hair) at the region of the detected style feature. In some examples, the parameter or multiple parameters may be determined over at least part of the extent of the subject's head. For example, a hair length may be determined at different positions on the subject's head. In this way, determining the parameter may be considered to comprise determining a local parameter (or multiple local parameters), or determining a parameter map over the head.

In the example where the style feature comprises a hair style feature, the step of determining a parameter may comprise performing an image segmentation technique to determine boundaries between regions of hair (e.g., a beard or moustache) and regions of skin, and in a further example, may determine boundaries between regions of hair of different lengths. Thus, the determined parameter may comprise hair length, where a hairless region is considered to have a hair length of 0mm. In this example where the style feature comprises a make-up style feature, the step of determining a parameter may comprise detecting colors of different regions of the subject's head appearing in the spatial representation, for example to determine the nature and amount of make-up applied at different regions. Image segmentation techniques may be applied to detect boundaries between other regions in the spatial representation, and other parameters of various style features may be determined at step 108.
The steps 106 and 108 may, in some examples, be performed together or concurrently, as part of the same process. For example, detecting a style feature may be performed implicitly when a parameter of the style feature is determined.

In some embodiments, one or more machine learning algorithms may be used to detect features in the image data and/or in the spatial representation. Artificial neural networks can be particularly effective at object detection in images, and, in one example, a U-net neural network model may be used for performing image segmentation of the spatial representation.

Following the parameter detection performed at step 108, a "map" of the subject's head may be created indicating the parameters of the style features at various regions of the spatial representation.

The method 100 comprises, at step 110, generating a symmetrised spatial representation comprising at least one of: a symmetrised first side that is a symmetrised version of the second side of the spatial representation; and a symmetrised second side that is a symmetrised version of the first side of the spatial representation. In other words, at step 110, a mirrored version of the spatial representation is created by flipping the spatial representation about a line of symmetry extending vertically through the spatial representation (e.g., through the subject's head), such that the left side of the spatial representation and the right side of the spatial representation are effectively switched. The symmetrised spatial representation may be considered to be a symmetrised version of the spatial representation (or part thereof). In some examples, only one side of the spatial representation of the head/face may be symmetrised, for example if the subject has performed the personal care activity on one side of their head, and merely wants to determine how the other side of the head should be treated in order to achieve a symmetrical look. This may reduce the amount of processing that is performed. In other examples, however, both sides of the spatial representation may be symmetrised. The creation of the symmetrised spatial representation may be achieved using a symmetric mapping technique applied to the model template to which the received image data is mapped, whereby for each point on one side (e.g., the first side) of the model template, there exists a registered point on the opposite side (e.g., the second side). For example, there may be a correspondence or registration between the corners of the subject's mouth and/or the corners of the subject's eyes on opposite sides of the subject's face. Thus, the same symmetric mapping will be applied to the spatial representation of the subject's head.

At step 112, the method 100 comprises comparing the parameter of the style feature in the symmetrised spatial representation with the parameter of the style feature in the spatial representation. In other words, the parameter of the style feature determined at step 108 for a particular region on one side of the subject's head is compared with the parameter of the same style feature as it appears in the symmetrised spatial representation (i.e., on the opposite side of the subject's head).

The method 100 further comprises, at step 114, generating, based on the comparison, a delta representation for presentation to the subject, the delta representation including an indication of a difference between the parameter of the style feature in the symmetrised spatial representation with the parameter of the style feature in the spatial representation. The delta representation may be considered to be a representation showing the difference between the spatial representation and the symmetrised spatial representation. In other words, the delta representation shows the difference between the appearance of the subject's head as captured in the image data and the appearance of the subject's head after the symmetric mapping has been applied to symmetrise the spatial representation. This enables the subject to see what action needs to be taken in order to achieve a symmetrical styling effect on their head.

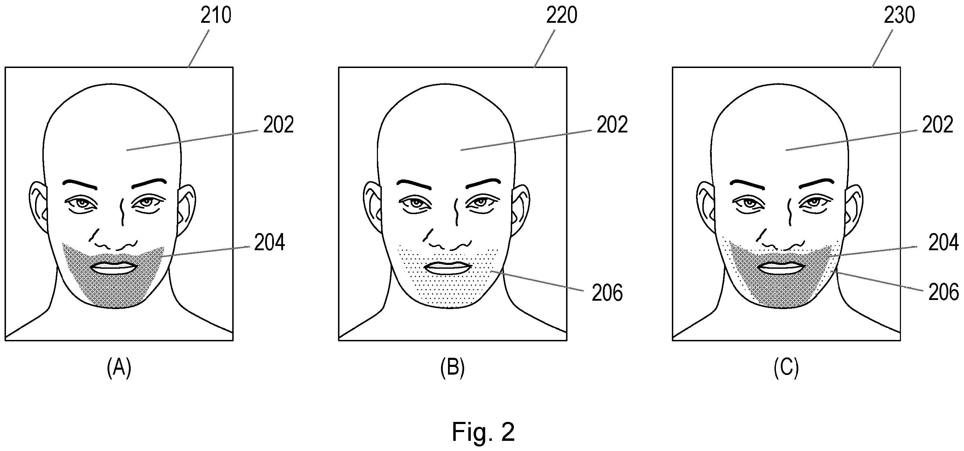

Fig. 2 is a collection of images showing representations that may be presented to a subject as part of the method 100. Fig. 2A shows a representation 210 of a head of a subject 202 having facial hair 204. The representation shown in Fig. 2A may, for example, be generated using the spatial representation of the subject's head, created at step 104 of the method 100. Fig. 2B shows a representation 220 of part of the head of the subject 202. In the representation 220, image segmentation processing has taken place to determine boundaries between regions in which the facial hair 204 has been detected and regions in which no facial hair has been detected. In this example, the regions 206 in which the facial hair 204 has been detected are indicated with hatching. The representation 220 is an example of the output of step 106 of the method 100. In order to generate the symmetrised spatial representation (i.e., step 110 of the method 100), a symmetry mapping may be applied to the representation 220 in order to create a symmetrised version of the left side of the subject's head and a symmetrised version of the right side of the subject's head. Fig. 2C shows a representation 230 of the head of the subject 202 and, in this representation, a symmetrised version of the hatched region 206 is indicated in addition to the facial hair 204 of the subject. Note that, as the subject continues to shave the facial hair, the representation 230 may be updated to show the current facial hair style. Thus, in this example, the facial hair 204 in the representation 230 differs slightly from the facial hair shown in the representation 210. The representation 230 is an example of the delta representation generated at step 114 of the method 100. With this representation 230, the subject is able to see the differences between their current facial hair style and a symmetric facial hair style. As the personal care activity proceeds (e.g., as the subject trims or shaves facial hair in regions corresponding to the hatched portions in the representation 230), the difference (e.g., the delta) between the actual facial hair style and the symmetric facial hair style reduces, until the difference ideally reaches a minimum.

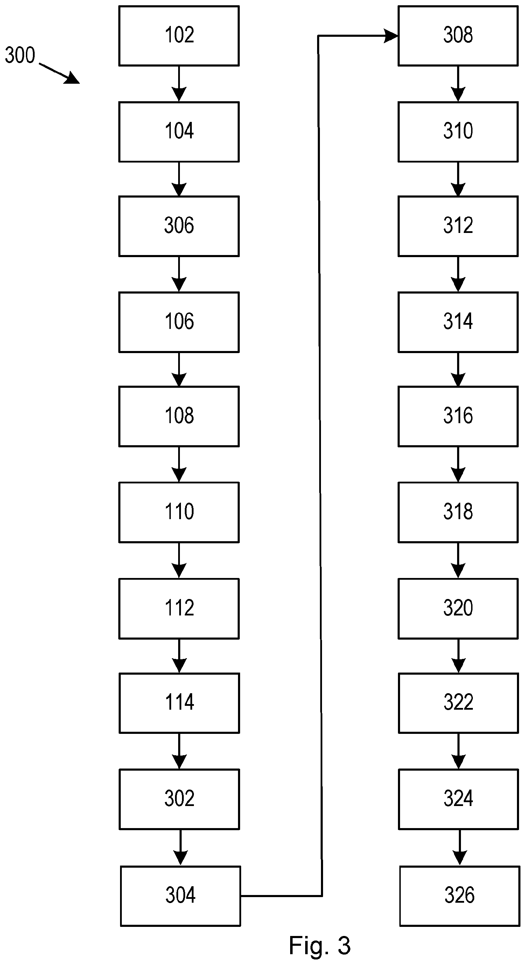

Fig. 3 is a flowchart of a further example of a method 300, such as a method for assisting a subject to perform a personal care activity. As with the method 100 discussed above, one or more steps of the method 300 may be performed using a processor or multiple processors. The method 300 may comprise, at block 302, providing the delta representation for presentation on a representation of the head of the subject. The delta representation may be presented as an overlay (e.g., superimposed) on the representation of the head of the subject. The representation of the head of the subject may comprise a representation selected from a group comprising: an image formed using the received image data; an avatar; a generic image of a head; and a reflection of the head of the subject. The presented images (e.g., the representation of the subject's head and the delta representation) may be presented on a display screen, such as the display of a computing device or an interactive mirror.

According to a further embodiment of the present disclosure, a subject may be provided with guidance of how to achieve a symmetrical look from the personal care activity. At step 304, the method 300 may further comprise generating a guidance indicator for presentation to the subject, the guidance indicator comprising an indication to guide the subject with regard to the performance of the personal care activity so as to reduce the difference between the parameter of the style feature in the symmetrised spatial representation with the parameter of the style feature in the spatial representation. In other words, the guidance generated at step 304 may be intended to assist the subject in reducing the difference between the actual style (e.g., facial hair style) and a symmetrical style to a minimum. The guidance indicator may be presented along with the representation of at least part of the subject's head and the delta representation, for example on a display screen. As such, the guidance indicator may comprise a visual indicator in some embodiments. In other embodiments, the guidance indicator may comprise an audible indicator, presented for example using a speaker. In examples in which the guidance indicator is a visual indicator, the guidance indicator may comprise textual instructions of where and how the personal care activity should be performed, a static graphical indicator, for example indicating regions where the subject should perform the personal care activity in order to achieve a more symmetrical look, or a dynamic graphical indicator, for example showing the subject in a dynamic way what further steps should be taken in the personal care activity to achieve the desired effect.

In one specific example, the style feature may comprise a feature relating to a style of hair present on the subject's face or head. For example, the style feature may comprise a style of facial hair. In such an example, the personal care activity may comprise a hair cutting activity, such as trimming or shaving the facial hair. Thus, the guidance indicator in this example may comprise an indication to guide the subject to cut the hair in a particular way. Some facial hair styles may include facial hair of a range of different lengths, depending on the location of the facial hair on the subject's face. In such examples, the guidance indicator may comprise an indication to guide the subject to cut the hair in order of decreasing intended hair length. For example, the subject may be presented with guidance (e.g., instructions) to trim regions having the longest hair (e.g., 4 mm) first, then presented with guidance to trim regions having the next longest hair (e.g., 2 mm), and finally presented with guidance to shave regions having the shortest hair (e.g., 0 mm or clean-shaven).

In some examples, the representation of the subject's head may be divided into zones, and the guidance presented to the subject may include guidance to perform the personal care activity in respect of one's own at a time. Once the subject has performed the personal care activity in respect of the part of their head corresponding to a particular zone, the guidance may change so as to guide the subject to move to the next zone.

As noted above, image segmentation may be used in order to determine the boundaries between regions of the subject's head having one style (e.g., trimmed facial hair) from regions of the subject's head having another style (e.g., clean-shaven). The process of image segmentation may be performed, in some embodiments, as part of the detecting step (step 106) or the determining step (step 108). In other embodiments, the image segmentation may be performed as part of a separate step in the method 300. For example, at step 306, the method 300 may further comprise performing image segmentation in respect of the received image data to determine a first style feature and a second style feature. The step of determining a parameter of the style feature (step 108) may comprise determining a style feature of each of the first style feature and the second style feature. It will be appreciated that the image segmentation may, in other examples, determine additional (e.g., third, fourth, and so on) style features.

In some examples, the methods 100, 300 disclosed herein may be performed using just one image or set of images of the subject's head. For example, the image data may be captured when the subject decides that they would like to receive assistance with the personal care activity, such as when the subject has performed the personal care activity on one half of their head. In other embodiments, however, steps of the method 100, 300 may be repeated, enabling the delta representation to be updated as the subject performs more of the personal care activity. For example, as the subject trims their facial hair, additional images of the subject's face that may be captured and the additional image data may be processed according to the methods disclosed herein in order to determine changes that have been made to the subject's face as a result of the personal care activity.

Thus, the method 300 may comprise, at step 308, receiving further image data relating to at least part of the subject's head. The further image data (e.g., one or more additional photographs) may be received after the image data received at step 102. At step 310, the method 300 may comprise updating the spatial representation of the subject's head based on the further image data. Thus, the further image data may be mapped into the model template onto the spatial representation created at step 104. The method 300 may further comprise, at step 312, detecting the style feature in the updated spatial representation, for example in a manner similar to the style feature detection made at step 106. The method 300 may then comprise, at step 314, determining an updated parameter of the style feature. For example, if a region of facial hair has been trimmed since the first image data was captured at step 102, then the parameter (e.g., the hair length) is updated to indicate the new hair length. At step 316, the method 300 may comprise generating an updated symmetrised spatial representation. Generating the updated symmetrised spatial representation may be achieved in a similar way as for step 110. Following the generation of the updated symmetrised spatial representation, the method 300 may comprise, at step 318, comparing the updated parameter of the style feature in the symmetrised spatial representation with the updated parameter of the style feature in the updated spatial representation. The comparison performed at step 318 catches any changes made to the parameters of the style features (e.g., any changes in hair length or make-up application) between receipt of the image data (step 102) and the further image data (step 308). At step 320, the method 300 may further comprise generating, based on the comparison made at step 318, an updated representation for presentation to the subject, the updated representation including an indication of a difference between the updated parameter of the style feature in the symmetrised spatial representation with the updated parameter of the style feature in the updated spatial representation. In other words, the delta representation is refreshed, so that the subject is able to see the latest comparison between the captured image data and the intended (e.g., symmetrical) version of the style feature. If steps 308 to 318 are repeated rapidly (e.g., by capturing further, updated image data every 1 second, half a second, 0.1 seconds, or even quicker), then the updated representation may be presented to with an appearance of the representation being refreshed almost in real time.

In some embodiments, in addition to providing a representation of the subject's head for presentation, an indication of the personal care device being used to perform the personal care activity may also be presented. In order to display the personal care device (or a representation thereof) in an accurate position relative to the subject's head, there is embodiments of the methods may involve determining the location of the personal care device. Thus, at step 322, the method 300 may comprise receiving location data indicative of a location of a personal care device used to perform the personal care activity. The method 300 may further comprise, at step 324, providing an indication of the location of the personal care device for presentation with the representation to the subject. By presenting an indication of the location of the personal care device along with the representation of the subject's head, the subject is able to understand where the personal care device is relative to the region of their head that is to be treated in order to achieve asymmetrical style feature. It may, for example, be difficult for the subject to see on their own head where a treatment head of the personal care device is located, and this may also be difficult to view in a reflection of the subject's head if, for example, their hand or another part of their body obstructs the subject's view of the personal care device.

The location of the personal care device may be determined in a number of ways, as discussed in greater detail below.

In some embodiments, the method 300 may involve controlling the personal care device in order to aid the subject to perform the personal care activity. Thus, at step 326, the method 300 may comprise generating an instruction signal for delivery to a personal care device used to perform the personal care activity, the instruction signal configured to control an operating parameter of the personal care device to assist the user of the personal care device to reduce the difference between the parameter of the style feature in the symmetrised spatial representation with the parameter of the style feature in the spatial representation. For example, an instruction signal may be generated and delivered to a personal care device (e.g., a hair cutting device) that automatically causes the hair cutting device to switch off its power when it is determined that an intended level of symmetry has been achieved. In another example, an instruction signal may be generated causing a hair cutting device to adjust its cutting length in order to achieve a symmetrical facial hairstyle of appropriate hair length.

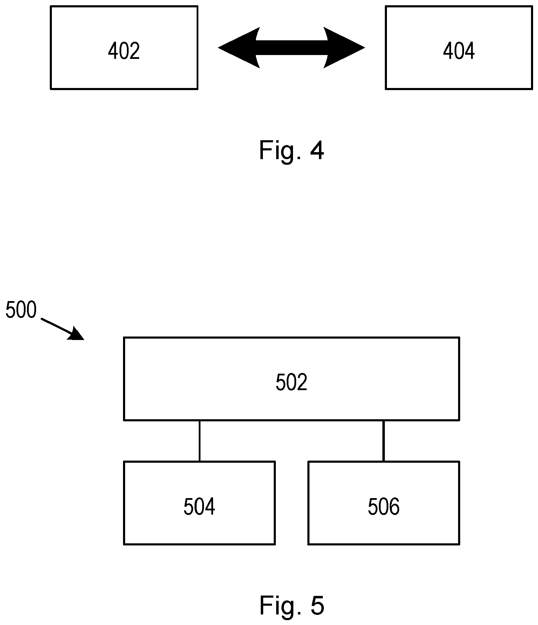

According to a further aspect, the present invention provides a computer program product. Fig. 4 is a schematic illustration of an example of a processor 402 in communication with a computer-readable medium 404. The computer program product comprises a non-transitory computer-readable medium 404, the computer-readable medium having computer-readable code embodied therein, the computer-readable code being configured such that, on execution by a suitable computer or processor 402, the computer or processor is caused to perform steps of the methods 100, 300 disclosed herein.

According to a further aspect, the present invention provides a system. Fig. 5 is a schematic illustration of an example of a system 500, such as a system for assisting a subject to perform a personal care activity. The system 500 may, for example, be used to perform the methods 100, 300 disclosed herein. The system 500 comprises a processor 502, an imaging device 504 configured to capture image data relating to at least part of a subject's head and a display 506. The processor 502 is configured to perform steps of the methods 100, 300. For example, the processor 502 is configured to receive, from the imaging device, image data relating to at least part of a subject's head; create a spatial representation of the subject's head by mapping the received image data to a model template having a first side and a second side; detect a style feature in the spatial representation; determine a parameter of the style feature; generate a symmetrised spatial representation comprising at least one of: a symmetrised first side that is a symmetrised version of the second side of the spatial representation; and a symmetrised second side that is a symmetrised version of the first side of the spatial representation; compare the parameter of the style feature in the symmetrised spatial representation with the parameter of the style feature in the spatial representation; and generate, based on the comparison, a representation for presentation to the subject via the display, the representation including an indication of a difference between the parameter of the style feature in the symmetrised spatial representation with the parameter of the style feature in the spatial representation.

Components of the system 500 may form part of the single unit (e.g., a computing device, a smart phone, or an interactive mirror) or separate components that may be connected to one another. In one example, components of the system 500 may comprise components of a smart phone, and functionality performed by the processor 502 may form part of a smart phone application.

In some embodiments, the imaging device 504 may be further configured to capture image data relating to a personal care device used to perform the personal care activity. For example, the personal care device may appear in the image data captured in respect of the subject's head. The processor 502 may be configured to determine, based on the captured image data relating to a personal care device, a location of a personal care device. The location of the personal care device may be determined relative to the head of the subject and/or relative to one or more other reference points. The processor 502 may be further configured to provide an indication of the location of the personal care device for presentation with the representation to the subject via the display 506. For example, the indication of the personal care device may be represented by a dot, a line, a symbol, a representation of the personal care device or an image of the personal care device as captured by the imaging device 504.

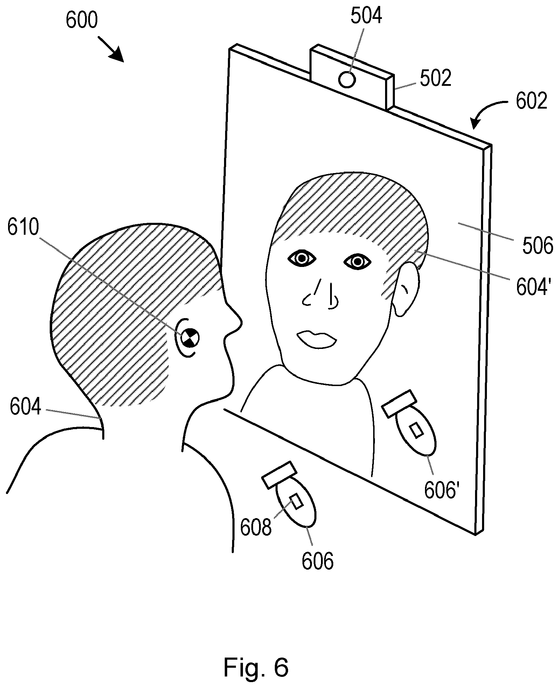

Fig. 6 is an illustration of a further example of a system 600 according to various embodiments. The system 600 includes the processor 502, the imaging device 504 and the display 506 which, in this example, the display 506 forms part of an interactive mirror 602. An interactive mirror, sometimes referred to as a smart mirror, is a unit which, in addition to functioning as a mirror to show a user (e.g. the subject) their reflection, is also capable of displaying information to the user. Information, such as text, images and videos, may be displayed on a display portion of the interactive mirror which may, for example, be positioned behind a mirrored (or partially mirrored) panel or a mirrored (or partially mirrored) surface. In this way, the display screen, or portions thereof, may be visible through the mirror portion, so that a user is able to simultaneously view their reflection and information presented on the display screen. The interactive mirror 602 includes the imaging device 504 in the form of a camera capable of capturing an image (e.g., of a subject 604) which can be displayed on the display 506 of the interactive mirror or used for determining data (e.g., device location data) as explained below. The interactive mirror 602 may be connected (wirelessly or via a wired connection) to one or more other devices, such as a personal care device 606. The processor 502 within the interactive mirror 602 may receive data from the personal care device 606 (and/or from other connected devices) and may display information to the subject relating to the connected device.

In this example, the subject 604 is able to view their reflection 604' in the interactive mirror 602, along with a reflection 606' of the personal care device 606. In examples where the display 506 comprises a display of a device other than an interactive mirror, a representation of the subject 604 and/or the personal care device 606 may be generated and presented to the subject.

Thus, as shown in Fig. 6, the system 600 may further comprise the personal care device 606 for applying style features to the subject's head, the personal care device used to perform the personal care activity.

As noted above, the location of the personal care device 606 may in some embodiments be determined using the image data acquired by the imaging device 504. In other embodiments, other components may be used to enable a determination of the location to be made. For example, in one embodiment, the personal care device 606 may comprise a sensor 608 configured to measure at least one motion parameter indicative of a motion of the personal care device. The sensor 608 may comprise one or more of an accelerometer, a gyroscope, a proximity sensor, a magnetometer, an optical sensor and an inertial measurement unit (IMU). The processor 502 may be configured to determine a location of a personal care device based on the measured motion parameter.

In another example, the location of the personal care device may be determined with reference to another device. Thus, the system 600 may further comprise a location beacon 610 configured to be worn by the subject. The location beacon 610 may be incorporated into a wearable device, such as a device to be worn in the subject's ear. The location beacon 610 may include components to communicate with the personal care device 606, enabling a location of the personal care device to be determined relative to the location beacon. The processor 502 may be configured to determine a location of the personal care device 606 relative to the location beacon 610. In some examples, multiple techniques for determining the location of the personal care device 606 may be used.

While it will be clear from the above disclosure that components of the system 600 may take many forms, in one non-limiting example, the personal care device 606 may comprise a hair cutting device, such as a hair clipper, a hair trimmer, or a shaver. The imaging device 504 and the display 506 may comprise components of at least one of a personal electronic device (e.g., a smart phone or a tablet computer) and an interactive mirror, as in the example discussed above. The personal care activity may comprise a hair cutting activity (e.g., trimming or shaving one's facial hair). In such examples, the parameter of the style feature may comprise a length of the hair on the subject's head.

Embodiments disclosed herein provide a mechanism by which a subject is provided with an indication of how a particular styling effect on their head (e.g., facial hair style or make up) compares to a symmetric style. This makes it easier for the subject to perform a personal care activity in such a way that a symmetrical style is achieved.

The processor 402, 502 can comprise one or more processors, processing units, multicore processors or modules that are configured or programmed to control the components of the system 500, 600 in the manner described herein. In particular implementations, the processor 402, 502 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g., Java interpreter instructions). Alternatively, one or more or all of the sub-routines may be stored in at least one external library file and linked with a main program either statically or dynamically, e.g., at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (100) for assisting a subject to perform a personal care activity, the method comprising:
receiving (102) image data relating to at least part of a subject's head
creating (104) a spatial representation of the subject's head by mapping the received image data to a model template having a first side and a second side;
detecting (106) a style feature in the spatial representation;
determining (108) a parameter of the style feature;
generating (110) a symmetrised spatial representation comprising at least one of: a symmetrised first side that is a symmetrised version of the second side of the spatial representation; and a symmetrised second side that is a symmetrised version of the first side of the spatial representation;
comparing (112) the parameter of the style feature in the symmetrised spatial representation with the parameter of the style feature in the spatial representation; and
generating (114), based on the comparison, a delta representation for presentation to the subject, the delta representation including an indication of a difference between the parameter of the style feature in the symmetrised spatial representation with the parameter of the style feature in the spatial representation.

2. A computer-implemented method (100, 300) according to claim 1, further comprising:
providing (302) the delta representation for presentation on a representation of the head of the subject;
wherein the representation of the head of the subject comprises a representation selected from a group comprising: an image formed using the received image data; an avatar; a generic image of a head; and a reflection of the head of the subject.

3. A computer-implemented method (100, 300) according to claim 1 or claim 2, further comprising:
generating (304) a guidance indicator for presentation to the subject, the guidance indicator comprising an indication to guide the subject with regard to the performance of the personal care activity so as to reduce the difference between the parameter of the style feature in the symmetrised spatial representation with the parameter of the style feature in the spatial representation.

4. A computer-implemented method (100, 300) according to claim 3, wherein the style feature comprises a feature relating to a style of hair present on the subject's head;
wherein the personal care activity comprises a hair cutting activity; and
wherein the guidance indicator comprises an indication to guide the subject to cut the hair in order of decreasing intended hair length.

5. A computer-implemented method (100, 300) according to any of the preceding claims, further comprising:
performing (306) image segmentation in respect of the received image data to determine a first style feature and a second style feature;
wherein determining a parameter of the style feature comprises determining a style feature of each of the first style feature and the second style feature.

6. A computer-implemented method (100, 300) according to any of the preceding claims, further comprising:
receiving (308) further image data relating to at least part of the subject's head;
updating (310) the spatial representation of the subject's head based on the further image data;
detecting (312) the style feature in the updated spatial representation;
determining (314) an updated parameter of the style feature;
generating (316) an updated symmetrised spatial representation;
comparing (318) the updated parameter of the style feature in the symmetrised spatial representation with the updated parameter of the style feature in the updated spatial representation; and
generating (320), based on the comparison, an updated representation for presentation to the subject, the updated representation including an indication of a difference between the parameter of the style feature in the symmetrised spatial representation with the parameter of the style feature in the spatial representation.

7. A computer-implemented method (100, 300) according to any of the preceding claims, further comprising:
receiving (322) location data indicative of a location of a personal care device used to perform the personal care activity; and
providing (324) an indication of the location of the personal care device for presentation with the representation to the subject.

8. A computer-implemented method (100, 300) according to any of the preceding claims, further comprising:
generating (326) an instruction signal for delivery to a personal care device used to perform the personal care activity, the instruction signal configured to control an operating parameter of the personal care device to assist the user of the personal care device to reduce the difference between the parameter of the style feature in the symmetrised spatial representation with the parameter of the style feature in the spatial representation.

9. A computer program product comprising a non-transitory computer-readable medium (404), the computer-readable medium having computer readable code embodied therein, the computer-readable code being configured such that, on execution by a suitable computer or processor (402), the computer or processor is caused to perform the method (100, 300) of any of the preceding claims.

10. A system (500) for assisting a subject to perform a personal care activity, the system comprising:
an imaging device (504) configured to capture image data relating to at least part of a subject's head;
a display (506); and
a processor (502) configured to:
receive, from the imaging device, image data relating to at least part of a subject's head;
create a spatial representation of the subject's head by mapping the received image data to a model template having a first side and a second side;
detect a style feature in the spatial representation;
determine a parameter of the style feature;
generate a symmetrised spatial representation comprising at least one of: a symmetrised first side that is a symmetrised version of the second side of the spatial representation; and a symmetrised second side that is a symmetrised version of the first side of the spatial representation;
compare the parameter of the style feature in the symmetrised spatial representation with the parameter of the style feature in the spatial representation; and
generate, based on the comparison, a representation for presentation to the subject via the display, the representation including an indication of a difference between the parameter of the style feature in the symmetrised spatial representation with the parameter of the style feature in the spatial representation.

11. A system (500, 600) according to claim 10, wherein the imaging device (504) is further configured to capture image data relating to a personal care device used to perform the personal care activity;
wherein the processor (502) is configured to:
determine, based on the captured image data relating to a personal care device, a location of a personal care device; and
provide an indication of the location of the personal care device for presentation with the representation to the subject via the display.

12. A system (500, 600) according to claim 10 or claim 11, further comprising:
a personal care device (606) for applying style features to the subject's head, the personal care device used to perform the personal care activity.

13. A system (500, 600) according to claim 12, wherein the personal care device comprises a sensor (608) configured to measure at least one motion parameter indicative of a motion of the personal care device;
wherein the processor (502) is configured to:
determine a location of a personal care device based on the measured motion parameter.

14. A system (500, 600) according to claim 12, further comprising:
a location beacon (610) configured to be worn by the subject;
wherein the processor (502) is configured to:
determine a location of the personal care device relative to the location beacon.

15. A system (500, 600) according to any of claims 12 to 14, wherein the personal care device (606) comprises a hair cutting device;
wherein the imaging device (504) and the display (506) are components of at least one of: a personal electronic device; and an interactive mirror (602);
wherein the personal care activity comprises a hair cutting activity; and
wherein the parameter of the style feature comprises a length of hair on the subject's head.
